(19) Europäisches Patentamt / European Patent Office / Office européen des brevets

(11) **EP 4 108 747 B1**

(12) **EUROPEAN PATENT SPECIFICATION**

(45) Date of publication and mention
of the grant of the patent:
**08.05.2024  Bulletin 2024/19**

(21) Application number: **22179620.4**

(22) Date of filing: **17.06.2022**

(51) International Patent Classification (IPC):
**C11B 1/02** *(2006.01)*      **C11B 1/06** *(2006.01)*

(52) Cooperative Patent Classification (CPC):
**C11B 1/025; C11B 1/06**

(54) **PROCESS FOR PRODUCING OLIVE OIL**

VERFAHREN ZUM PRODUZIEREN VON OLIVENÖL

PROCÉDÉ DE PRODUCTION D'HUILE D'OLIVE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.06.2021  EP 21181065
07.12.2021  EP 21212805**

(43) Date of publication of application:
**28.12.2022   Bulletin 2022/52**

(73) Proprietor: **DSM IP Assets B.V.
6411 TE  Heerlen (NL)**

(72) Inventors:
• **ZHA, Ying**
**6100 AA Echt (NL)**
• **GUILONARD, Lambertus Jacobus Otto**
**6100 AA Echt (NL)**

(74) Representative: **dsm-firmenich IP
P.O. Box 4
6100 AA Echt (NL)**

(56) References cited:
**DE-A1- 10 339 010      US-B1- 6 399 347**

• HADJ-TAIEB NOOMEN ET AL: "Optimisation of
olive oil extraction and minor compounds
content of Tunisian olive oil using enzymatic
formulations during malaxation", BIOCHEMICAL
ENGINEERING JOURNAL, vol. 62, 20 April 2011
(2011-04-20), pages 79-85, XP028905466, ISSN:
1369-703X, DOI: 10.1016/J.BEJ.2011.04.003
• OBERGFOELL H-M: "THE USE OF ENZYMES IN
THE EXTRACTION", RIFT APPLICABILITY;
DRAFT-IETF-RIFT-APPLICABILITY-00.TXT;
INTERNET-DRAFT: RIFT WG, INTERNET
ENGINEERING TASK FORCE, IETF;
STANDARDWORKINGDRAFT, INTERNET
SOCIETY (ISOC) 4, RUE DES FALAISES CH- 1205
GENEVA, SWITZERLAND, vol. 4, no. 1, 1 January
1997 (1997-01-01) , pages 35-37, XP000683696,
ISSN: 1258-8210
• CHIACCHIERINI ET AL: "Impact evaluation of
innovative and sustainable extraction
technologies on olive oil quality", TRENDS IN
FOOD SCIENCE AND TECHNOLOGY, ELSEVIER
SCIENCE PUBLISHERS, GB, vol. 18, no. 6, 10 May
2007 (2007-05-10), pages 299-305, XP022069624,
ISSN: 0924-2244, DOI:
10.1016/J.TIFS.2007.01.008

**Description**

**Field of the invention**

The present invention generally relates to the fields of enzymology and industrial olive oil production. Particularly, the present invention relates to processes for producing olive oil. More particularly, the present invention relates to enzymes and their use in processes for producing olive oil.

**Background of the invention**

In the last 30 years total worldwide consumption of olive oil almost doubled. Olive oil is not only used in the food industry due to its high amount of health promoting constituents such as antioxidants and monounsaturated fatty acids but is also widely used in other industries such as the pharmaceutical, cosmetic, soap producing, textile and lubricant producing industry.

For many years conventional techniques have been used in olive oil extraction. However, increasing demand for high quality olive oil at the highest yield and minimum cost as well as using environmentally friendly olive oil production processes have urged olive oil producers to develop and adopt novel technologies for olive oil extraction such as ultrasound, pulsed electric field or microwave technology. DE1 033901 0A1 discloses a process for obtaining oil from olives involving the use of enzymes.

Despite the progress in olive oil production technology, the quality of the obtained oil and the extraction yield can still be further optimized. Therefore, still a need remains in the art to provide improved processes for olive oil production.

**Summary of the invention**

An object of the invention is a process for producing olive oil by using enzymes. Optimization and improvement lie *inter alia* in the enzymes used in the process.

**Detailed description of the invention**

Throughout the present specification and the accompanying claims, the words "comprise" and "include" and variations such as "comprises", "comprising", "includes" and "including" are to be interpreted inclusively. That is, these words are intended to convey the possible inclusion of other elements or integers not specifically recited, where the context allows. The articles "a" and "an" are used herein to refer to one or to more than one (i.e. to one or at least one) of the grammatical object of the article. By way of example, "an element" may mean one element or more than one element. Any recited process can be carried out in the order of events recited or in any other order which is logically possible.

An aspect of the invention relates to a process for producing olive oil comprising the step of adding enzymes to olive paste and incubating the obtained mixture, wherein the enzymes comprise a polygalacturonase, a pectin methyl esterase, a pectin lyase, an arabinofuranosidase, a cellulase, a xylanase, a rhamnogalacturonase and a mannanase.

As used herein, a polygalacturonase (EC 3.2.1.15) is any polypeptide which is capable of catalysing the random hydrolysis of 1,4-$\alpha$-D-galactosiduronic linkages in pectate and other galacturonans. This enzyme may also be referred to as endo-polygalacturonase, pectin depolymerase, pectinase, endopolygalacturonase, pectolase, pectin hydrolase, pectin polygalacturonase, poly-$\alpha$-1,4-galacturonide glycanohydrolase, endogalacturonase; endo-D-galacturonase or poly(1,4-$\alpha$-D-galacturonide) glycanohydrolase.

As used herein, a pectin methyl esterase (EC 3.1.1.11) is any enzyme which is capable of catalysing the reaction: pectin + n $H_2O$ = n methanol + pectate. The enzyme may also be known as pectinesterase, pectin demethoxylase, pectin methoxylase, pectin methylesterase, pectase, pectinoesterase or pectin pectylhydrolase.

As used herein, an pectin lyase (EC 4.2.2.10) is any enzyme capable of catalysing the eliminative cleavage of (1→4)-$\alpha$-D-galacturonan methyl ester to give oligosaccharides with 4-deoxy-6-O-methyl-$\alpha$-D-galact-4-enuronosyl groups at their non-reducing ends. The enzyme may also be known as endo-pectin lyase, pectin trans-eliminase, endo-pectin lyase, polymethylgalacturonic transeliminase, pectin methyltranseliminase, pectolyase, PL, PNL or PMGL or (1→4)-6-O-methyl-$\alpha$-D-galacturonan lyase.

As used herein, an arabinofuranosidase (EC 3.2.1.55) is any polypeptide which is capable of acting on $\alpha$-L-arabinofuranosides, $\alpha$-L-arabinans containing (1,2) and/or (1,3)- and/or (1,5)-linkages, arabinoxylans and arabinogalactans. This enzyme may also be referred to as $\alpha$-N-arabinofuranosidase, $\alpha$-L-arabinofuranosidase or arabinosidase. Examples of arabinofuranosidases include, but are not limited to, arabinofuranosidases from *Aspergillus niger, Humicola insolens* DSM 1800 (see WO 2006/114094 and WO 2009/073383) and *M. giganteus* (see WO 2006/114094).

As used herein, a cellulase is any polypeptide which is capable of degrading or modifying cellulose. Cellulases break down the cellulose molecule into monosaccharides or shorter polysaccharides and oligosaccharides. Examples of cellulases include, but are not limited to, cellobiohydrolase, endoglucanase and beta-glucosidases.

As used herein, a beta-glucosidase (EC 3.2.1.21) is any polypeptide which is capable of catalysing the hydrolysis of terminal, non-reducing β-D-glucose residues with release of β-D-glucose. Such a polypeptide may have a wide specificity for β-D-glucosides and may also hydrolyze one or more of the following: a β-D-galactoside, an α-L-arabinoside, a β-D-xyloside or a β-D-fucoside. This enzyme may also be referred to as amygdalase, β-D-glucoside glucohydrolase, cellobiase or gentobiase.

As used herein, endoglucanases are enzymes which are capable of catalyzing the endohydrolysis of 1,4-β-D-glucosidic linkages in cellulose, lichenin or cereal β-D-glucans. They belong to EC 3.2.1.4 and may also be capable of hydrolyzing 1,4-linkages in β-D-glucans also containing 1,3-linkages. Endoglucanases may also be referred to as cellulases, avicelases, β-1,4-endoglucan hydrolases, β-1,4-glucanases, carboxymethyl cellulases, celludextrinases, endo-1,4-β-D-glucanases, endo-1,4-β-D-glucanohydrolases or endo-1,4-β-glucanases.

As used herein, a cellobiohydrolase (EC 3.2.1.91) is any polypeptide which is capable of catalyzing the hydrolysis of 1,4-β-D-glucosidic linkages in cellulose or cellotetraose, releasing cellobiose from the ends of the chains. This enzyme may also be referred to as cellulase 1,4-β-cellobiosidase, 1,4-β-cellobiohydrolase, 1,4-β-D-glucan cellobiohydrolase, avicelase, exo-1,4-β-D-glucanase, exocellobiohydrolase or exoglucanase.

As used herein, a xylanase belongs to EC 3.2.1.8 or EC 3.2.1.136. Xylanase belonging to EC 3.2.1.8 is any polypeptide which is capable of catalysing the endohydrolysis of 1,4-β-D-xylosidic linkages in xylans. This enzyme may also be referred to as endo-1,4-β-xylanase, endoxylanase or 1,4-β-D-xylan xylanohydrolase. Xylanase belonging to EC 3.2.1.136 is a glucuronoarabinoxylan endoxylanase, an enzyme that is able to hydrolyze 1,4 xylosidic linkages in glucuronoarabinoxylans.

As used herein, rhamnogalacturonase (EC 3.2.1.171) is any polypeptide which is capable of hydrolyzing the linkage between galactosyluronic acid and rhamnopyranosyl in an endo-fashion in strictly alternating rhamnogalacturonan structures, consisting of the disaccharide [(1,2-alpha-L-rhamnoyl-(1,4)-alpha-galactosyluronic acid]. The enzyme is also known as rhamnogalacturonan hydrolase and leads to endohydrolysis of the alpha-D-GalA-(1->2)-alpha-L-Rha glycosidic bond in the rhamnogalacturonan I backbone with initial inversion of anomeric configuration releasing oligosaccharides with beta-D-GalA at the reducing end.

As used herein, a mannanase (EC 3.2.1.78 or EC 3.2.1.25) is any polypeptide which is capable of catalysing the random hydrolysis of 1,4-β-D-mannosidic linkages in mannans, galactomannans and glucomannans. This enzyme may also be referred to as beta-mannanase, mannan endo-1,4-β-mannosidase or endo-1,4-mannanase.

In an embodiment the enzymes further comprise a beta-glucanase. As used herein, a beta-glucanase is an endoglucanase. Endoglucanases are enzymes which are capable of catalyzing the endohydrolysis of 1,4-β-D-glucosidic linkages in cellulose, lichenin or cereal β-D-glucans. They belong to EC 3.2.1.4 and may also be capable of hydrolyzing 1,4-linkages in β-D-glucans also containing 1,3-linkages. Endoglucanases may also be referred to as cellulases, avicelases, β-1,4-endoglucan hydrolases, β-1,4-glucanases, carboxymethyl cellulases, celludextrinases, endo-1,4-β-D-glucanases, endo-1,4-β-D-glucanohydrolases or endo-1,4-β-glucanases. In an embodiment the endoglucanase comprises a GH5 endoglucanase and/or a GH7 endoglucanase. In a preferred embodiment the endoglucanase comprises a GH5 endoglucanase. GH classification can be found on the CAZy website.

In an embodiment the enzymes may further comprise additional enzymes including, but not limited to, beta-xylosidase, lytic polysaccharide monooxygenase, glucuronidase, acetyl xylan esterase, feruloyl esterase, coumaroyl esterase, galactosidase, endo-galactanase, pectin acetyl esterase, pectate lyase, alpha-rhamnosidase, exo-galacturonase, exopolygalacturonate lyase, rhamnogalacturonan lyase, rhamnogalacturonan acetyl esterase, rhamnogalacturonan galacturonohydrolase, xylogalacturonase, endo-arabinanase, protease, lipase, ligninase, hexosyltransferase, amylase, glucoamylase, catalase, carbohydrase, carbonic anhydrase, carboxypeptidase, asparaginase, chitinase, chymosin, cutinase, cyclodextrin glycosyltransferase, deoxyribonuclease, epimerase, glucan lysase, glucose oxidase, glycosyl hydrolase, hexose oxidase, hydrolase, hyaluronic acid synthase, invertase, isomerase, laccase, lyase, mannosidase, mutanase, oxidase, oxidoreductase, pectinase, pectin depolymerase, pectinolytic enzymes, peptidase, perhydrolase, peroxidase, phenoloxidase, phytase, polyol oxidase, ribonuclease, transferase, transport protein, transglutaminase.

As described above, the enzymes comprise a polygalacturonase, a pectin methyl esterase, a pectin lyase, an arabinofuranosidase, a cellulase, a xylanase, a rhamnogalacturonase and a mannanase. The enzymes may comprise one or more of each of these enzymes. For example, the enzymes may comprise one or more polygalacturonases, i.e. a first polygalacturonase and a second polygalacturonase (which is different from the first). In case there are two or more polygalacturonases, they may origin from the same microorganism, *e.g.* fungus, or from different microorganisms, *e.g.* different fungi.

In an embodiment the enzymes are in liquid form. The enzymes can be added to the olive paste, but the olive paste may be also added to the enzymes. The enzymes and olive paste are contacted and then incubated. The enzymes may the added separately or combined as one or more compositions.

In an embodiment the enzymes are fungal enzymes. In an embodiment one or more of the enzymes are obtained from a fungus of the genus including, but not limited to, *Humicola, Rhizomucor, Myceliophthora, Rasamsonia, Talaromyces, Penicillium, Thermomyces, Thermoascus, Aspergillus, Scytalidium, Paecilomyces, Chaetomium, Stibella, Corynascus, Malbranchea* or *Thielavia.*

In an embodiment one or more of the enzymes are in the form of a whole fermentation broth. In other words, the whole fermentation broth may comprise one or more of the enzymes. The term "whole fermentation broth" as used herein refers to a preparation produced by cellular fermentation that undergoes no or minimal recovery and/or purification.

Generally, olive oil production begins with harvesting the olives. Olive picking is an important operation that affects virgin olive oil quality, since its sensory quality depends on the health and ripeness of the harvested olives. Traditionally, olives were hand-picked. Currently, harvesting is performed by a variety of types of shakers that transmit vibrations to the tree branches, causing the olives to drop into nets that have previously been placed under the tree canopy and then they are collected with vacuum systems. Increasing ripeness generally increases yield in terms of release of olives from the tree branches. However, over-mature olives do not possess the best sensory qualities for oil production. Therefore, harvesting time is frequently a compromise between harvesting efficiency and final oil quality.

After harvesting, the olives are transported to the olive mill. In any case, the harvested olives should be transported in such a way that air circulation prevents the rise of temperature on the bottom layers of olives. Regardless of the transport system, the olives should be stored under optimal conditions from the moment of harvesting up to their processing at the mill. The time between both events should be no longer than two days to avoid significant changes in the oil sensory profile of healthy and optimally ripe olives. The olives arriving at the olive mill do not have the same characteristics: there will be olives of various cultivars, at different levels of maturity and even in different sanitary conditions (e.g. infested by parasites), which demands the classification of batches according to pre-established quality levels. Thus, batches are weighed and later inspected by an expert who channels them to the reception lanes that match their quality. A sample of olives representative of the whole batch is randomly taken for several analyses: humidity, free acidity, yield and sensory assessment.

After arriving at the olive mill, the olives are washed to remove dirt, leaves and twigs, as these materials could be harmful to the olive mill machinery and they could also modify the sensory attributes of the extracted olive oil, e.g. the presence of leaves increases bitterness. The machinery consists of powerful fans, which remove foreign vegetable material, and pipes with forced water circulation in which olives are washed. After the dirt, leaves and twigs are filtered out, the olives are ready for processing into oil. They are put on a moving belt to the processing zone of the olive mill.

In an embodiment the enzymes are added during and/or after crushing olives into olive paste. The crushing process is important in order to guarantee the taste and aroma of the end product and also the yield of the extraction process. The term "crushing" as used herein also encompasses milling and grinding Traditional olive oil processing begins with crushing the olives into a paste. The purpose of the crushing is to facilitate the release of the oil from the vacuoles. The crushing can be done in a traditional stone mill having large granite stones. The mill may also be motorized including wiper blades. Modern olive mills are partially or fully automated and have replaced granite stones with metal crushers. They consist of a stainless-steel body and a stainless steel crusher that rotates at high speed. The olives are typically thrown against a hammer-shaped metal grating. Alternatives include toothed disc, cylinder, and roller mills. Modern milling is very gentle in order to avoid overheating of the paste. Cold pressed (extra virgin) oils must not exceed 27°C at any step in the processing of the oil.

In an embodiment the enzymes are added in an amount ranging from 100 to 1000 ppm. In an embodiment the enzymes are added in an amount ranging from 150 to 900 ppm. In an embodiment the enzymes are added in an amount ranging from 200 to 800 ppm, In an embodiment the enzymes are added in an amount ranging from 250 to 700 ppm. In an embodiment the enzymes are added in an amount ranging from 300 to 600 ppm.

Variety and maturation are two of the most important factors of olives that influence the quality and taste of the final olive oil. Maturation can be measured based on the maturity index.

In an embodiment the olive paste is made from olives having a maturity index of 0 or 1. When the olive paste is made from olives having a maturity index of 0 or 1, obviously also the olive oil produced from the olive paste is made from olives having a maturity index of 0 or 1.

In a preferred embodiment when the olive paste is made from olives having a maturity index of 0 or 1, the enzymes further comprise a beta-glucanase. The present application therefore also relates to a process for producing olive oil comprising the step of adding enzymes to olive paste made from olives having a maturity index of 0 or 1 and incubating the obtained mixture, wherein the enzymes comprise a polygalacturonase, a pectin methyl esterase, a pectin lyase, an arabinofuranosidase, a cellulase, a xylanase, a rhamnogalacturonase, a mannanase and a beta-glucanase.

In another embodiment the olive paste is made from olives having a maturity index of 2 or 3. When the olive paste is made from olives having a maturity index of 2 or 3, obviously also the olive oil produced from the olive paste is made from olives having a maturity index of 2 or 3.

The maturity index of olives can be calculated as follows. Firstly, take a random sample of about 0.91 kg (2 pounds) of olives from several trees of the same variety in the area where harvest is imminent. The olives should be selected from high and low in the trees and from all sides. All the olives from a branch should be collected here and there rather than individual olives. The olives can be collected in a sample bucket. Secondly, select 100 olives out of the sample bucket. Repeat this 3-10 times until all olives are gone. Thirdly, separate each 100 olives sample into 8 color categories based on the following color chart: 0 = deep-green skin, fruit hard; 1 = green-yellowish skin, fruit starting to soften; 2 = green skin with reddish spots (i.e. < 50% of the skin turning red, purple or black); 3 = reddish-brown skin (i.e. > 50% of the skin turning red, purple or black); 4 = purple or black skin with all white or green flesh; 5 = purple or black skin with <50% of the flesh turning purple; 6 = purple or black skin with >50% but <100% of the flesh turning purple; 7 = purple or black skin with purple flesh to the pit. Fourthly, multiply the number of olives in each color category by the number of that color category (0 to 7). Fifthly, add all the number together and divide by 100.

There are hundreds of varieties of olive trees. A wide range of olive varieties are used in the production of olive oil. These include Picual, Mission, Manzanillo, Sevillano, Arbequina, Tsunati, Koroneiki, Arbosana, Valanolia, Ascolano, Frantoio, Cailletier, Leccino, Pendolino, Maurino, and Coratina.

In an embodiment the process further comprises subjecting the obtained mixture to malaxation. As used herein, the term "malaxation" means mixing/stirring the obtained mixture. In general, metal crushers can cause emulsions that lower yield. The malaxation step diminishes this effect by causing the droplets produced by crushing the olives to coalesce, thereby increasing the percentage of available oil. Malaxation is a key step in the olive oil production process, as good malaxation means optimal oil extraction and good values of antioxidants and flavor.

In an embodiment the obtained mixture is incubated for 10 to 200 minutes at a temperature ranging from 5°C to 70°C. In an embodiment the obtained mixture is incubated for 20 to 100 minutes at a temperature ranging from 20°C to 30°C. Incubation may take place during malaxation. So, the incubation conditions will depend on the malaxation conditions of the respective olive oil production site.

The malaxers have two or three cylindrical vats in tandem, each one with a rotating helix with several wings that mix the paste at low speed (15-20 rpm) for a period of time, for example between 30 minutes and 75 minutes, depending on the paste characteristics. Longer mixing times increase yield, however, they may also result in increased oxidation and decreased shelf life and quality. The vats are made of stainless steel and have double walls for circulation of heated water. Temperature and time are the variables of the malaxation process that are automatically controlled. The extraction yield is directly correlated to these variables; a rise in temperature and time increases the yield. However, the temperature of the paste should not be higher than 30°C to avoid change in the oil color (from yellow-green to reddish), the increase of acidity and the degradation of the volatile compounds that would result in an olive oil with the negative sensory attribute "heated". New malaxers have an atmosphere controlled by an inert gas (i.e. nitrogen- or carbon dioxide-controlled atmosphere with around 2% oxygen content only) to reduce oxidation and produce higher-quality oils.

In an embodiment the obtained mixture is incubated in a container having a voume ranging from 0.5 m$^3$ to 5000 m$^3$. In an embodiment the container is a malaxer. The obtained mixture can be incubated in several containers (e.g. malaxers).

In an embodiment the process further comprises a solid-liquid separation after malaxation. In an embodiment the solid-liquid separation is performed by applying pressure, percolation and/or centrifugation techniques. The main constituents of olive paste are liquids, e.g. olive oil and water, and solids such as small pieces of kernel and tissues. The solid/liquid separation step is used to separate olive oil from the other constituents. As indicated above there are three different extraction processes that can be applied to do this: pressure, percolation and centrifugation.

The paste can be spread on fiber disks, which are stacked on top of each other, then placed into a press. The stacks of discs are then pressed in a hydraulic press. Pressure of up to 4,000 kPa is applied to the disks, compacting the solid phase and percolating the liquid phases (oil and water). Water can be used to run down the sides of the discs to increase the speed of percolation. The liquids are then separated by decantation or centrifugation.

Modern olive oil mills extract olive oil by means of centrifugation systems since they produce high-quality olive oils at lower production cost. The paste is pumped into an industrial decanter where the phases are separated using centrifugation. This step can involve a three-phase decanter or a two-phase decanter. Water can be added to facilitate the extraction process. The decanter is a large-capacity horizontal centrifuge rotating approximately 3,500 rpm. In three-phase decanting, inside the rotating conical drum is a coil that rotates more slowly than the drum. This pushes the solids out of one end of the system and the water and oil out the other end. Three-phase decanting results in loss of a portion of the oil polyphenolics due to the higher quantity of water used. It also produces larger quantities of olive mill wastewater that then need to be processed and have negative environmental effects. Two-phase decanters were created to solve these problems. In this process, the olive paste is separated into two phases, oil and wet pomace. The decanter has two exits instead of three, and the water is expelled with the pomace, resulting in a wetter pomace. The two-phase decanter thus solves the phenol washing issue and uses less water, but increases the waste produced.

Alternatively, the olive oil is extracted by means of the Sinolea method. In this process, rows of metal discs are dipped into the paste, and the oil preferentially sticks to the metal and is removed by scrapers in a continuous process. Based on the varying surface tension between the oil and vegetation water, the oil adheres to the steel plates while the other two phases remain behind. The Sinolea method continuously introduces hundreds of plates into the paste and is not very efficient, leaving lots of oil in the paste. The final paste still requires decanting.

Regardless of the process used for oil extraction, a final centrifugation process is needed to separate the oil from the vegetation water. Vertical centrifuges, operating at speeds of 6,000-7,000 rpm under controlled temperature conditions, are used for this purpose.

Following processing, the olive oil is stored in large stainless-steel tanks with nitrogen blanketing to protect it from oxygen. Preferably, the olive oil is stored at about 15-18°C. Although not necessary, virgin olive oil can be filtered prior to bottling. Diatomaceous earth is frequently used as a filter aid. Colored glass bottles are ideal packaging for olive oil, because the colored glass blocks the UV light and is also impermeable to oxygen. Bottling under nitrogen is a recommended practice.

The olive oil production process does not only give rise to olive oil, but several co-products are produced as well. These co-products contain healthy polyphenolics and fibers that can be beneficial to human health. Thus, new uses for the olive oil wastewater and their extracts are being explored as foods, supplements, and cosmetics. In addition, methods to stabilize olive pomace in an efficient manner are being studied, as are value-added final product uses for the pomace itself in foods.

Olive oils themselves exits in several grades including extra-virgin olive oil, lower grades of olive oil including virgin olive oil, refined olive oils and olive pomace oils.

Another aspect of the present invention relates to an olive paste comprising a polygalacturonase, a pectin methyl esterase, a pectin lyase, an arabinofuranosidase, a cellulase, a xylanase, a rhamnogalacturonase, a mannanase and optionally a beta-glucanase. All the features and embodiments of the process for producing olive oil as described above also apply to the olive paste as described herein.

## EXAMPLES

### Example 1

*Yield increase of olive oil production by using enzymes*

Olives of maturity index (MI) 1 are crushed into olive paste in a crusher. The olive paste obtained from the crusher is used directly in the trial.

Ten kilogram olive paste is incubated with and without an enzyme composition under slow stirring at ambient temperature (i.e. 25°C) for 20 to 40 minutes in a reaction vessel to produce olive oil. In experiment 1, no enzymes are used; in experiment 2, 300 ppm of an enzyme composition comprising a polygalacturonase, a pectin methyl esterase, a pectin lyase, an arabinofuranosidase, a cellulase and a xylanase is used; in experiment 3, 300 ppm of an enzyme composition comprising a rhamnogalacturonase and a mannanase is used; and in experiment 4, 150 ppm of an enzyme composition comprising a polygalacturonase, a pectin methyl esterase, a pectin lyase, an arabinofuranosidase, a cellulase and a xylanase and 150 ppm of an enzyme composition comprising a rhamnogalacturonase and a mannanase is used.

After incubation, the olive oil is separated from the olive pomace and water with a decanter centrifuge using the following settings: temperature setting is 30$^0$C. Throughput of the decanter: 20 L/h. Acceleration: 2000xg. The yield is determined and calculated by weighing the olive oil, olive pomace and water using the following formula:

$$\% \text{ Olive oil yield calculation} = (\text{Olive oil collected in Kg} / \text{input olive quantities in Kg}) * 100\%$$

Table 1: Olive oil yield increase

| Experiment number | Yield increase |
|---|---|
| 1 | - |
| 2 | + |
| 3 | + |
| 4 | +++ |

### Example 2

*Yield increase of olive oil production by using enzymes*

Olives of maturity index (MI) 1 are crushed into olive paste in a crusher. The olive paste obtained from the crusher is used directly in the trial.

Ten kilogram olive paste is incubated with and without an enzyme composition under slow stirring at ambient temperature (*i.e.* 25°C) for 20 to 40 minutes in a reaction vessel to produce olive oil. In experiment 1, no enzymes are used; in experiment 2, 300 ppm of an enzyme composition comprising a polygalacturonase, a pectin methyl esterase, a pectin lyase, an arabinofuranosidase, a cellulase and a xylanase is used; in experiment 3, 300 ppm of an enzyme composition comprising a rhamnogalacturonase and a mannanase is used; in experiment 4, 300 ppm of an enzyme composition comprising a beta-glucanase is used; in experiment 5, 150 ppm of an enzyme composition comprising a polygalacturonase, a pectin methyl esterase, a pectin lyase, an arabinofuranosidase, a cellulase and a xylanase and 150 ppm of an enzyme composition comprising a beta-glucanase is used; in experiment 6, 150 ppm of an enzyme composition comprising a rhamnogalacturonase and a mannanase and 150 ppm of an enzyme composition comprising a beta-glucanase is used; in experiment 7, 150 ppm of an enzyme composition comprising a polygalacturonase, a pectin methyl esterase, a pectin lyase, an arabinofuranosidase, a cellulase and a xylanase and 150 ppm of an enzyme composition comprising a rhamnogalacturonase and a mannanase is used; in experiment 8, 100 ppm of an enzyme composition comprising a polygalacturonase, a pectin methyl esterase, a pectin lyase, an arabinofuranosidase, a cellulase and a xylanase and 100 ppm of an enzyme composition comprising a rhamnogalacturonase and a mannanase and 100 ppm of an enzyme composition comprising a beta-glucanase is used.

After incubation, the olive oil is separated from the olive pomace and water with a decanter centrifuge using the following settings: temperature setting is 30°C. Throughput of the decanter: 20 L/h. Acceleration: 2000xg. The yield is determined and calculated by weighing the olive oil, olive pomace and water using the following formula:

$$\% \text{ Olive oil yield calculation} = (\text{Olive oil collected in Kg} / \text{input olive quantities in Kg}) * 100\%$$

Table 2: Olive oil yield increase

| Experiment number | Yield increase |
|---|---|
| 1 | - |
| 2 | + |
| 3 | + |
| 4 | + |
| 5 | ++ |
| 6 | ++ |
| 7 | +++ |
| 8 | +++++ |

### Example 3

*Yield increase of olive oil production by using enzymes*

Olives of maturity index (MI) 2.5 were crushed into olive paste in a crusher. The enzyme solution was added into the crusher during the entire crushing process.

One ton of crused olive paste was incubated with and without an enzyme composition under slow stirring at 25°C for 30 minutes in a malaxer. In experiment 1, no enzymes were used; in experiment 2, 250 ppm of an enzyme composition comprising a polygalacturonase, a pectin methyl esterase, a pectin lyase, an arabinofuranosidase, a cellulase and a xylanase was used; in experiment 3, 250 ppm of an enzyme composition comprising a rhamnogalacturonase and a mannanase was used; and in experiment 4, 125 ppm of an enzyme composition comprising a polygalacturonase, a pectin methyl esterase, a pectin lyase, an arabinofuranosidase, a cellulase and a xylanase and 125 ppm of an enzyme composition comprising a rhamnogalacturonase and a mannanase was used.

After incubation, the olive paste went into a decanter to separate the crude oil from the pomance, and the obtained crude oil went through a disc centrifuge to remove the water in the crude oil to obtain final olive oil product. During the decantation process, pomace was collected to measure the oil content by Near Infrared Reflectance (NIR), which was used as indicator of oil yield. The lower the oil content in the pomace, the higher the oil yield in the crude olive oil will be.

The results are shown in Table 3. Table 3 clearly demonstrates that there is a higher yield of olive oil when olive oil is produced by using a polygalacturonase, a pectin methyl esterase, a pectin lyase, an arabinofuranosidase, a cellulase, a xylanase, a rhamnogalacturonase and a mannanase compared to when olive oil is produced by using a polygalacturonase, a pectin methyl esterase, a pectin lyase, an arabinofuranosidase, a cellulase and a xylanase or when olive oil is produced by using a rhamnogalacturonase and a mannanase.

Table 3: Remaining olive oil in pomace after enzymatic treatment.

| Experiment number | Enzyme composition constituents | Dosage | Oil content in pomace |
|---|---|---|---|
| 1 | - | - | 13.12% |
| 2 | polygalacturonase, pectin methyl esterase, pectin lyase, arabinofuranosidase, cellulase and xylanase | 250 ppm | 6.06% |
| 3 | rhamnogalacturonase and mannanase | 250 ppm | 6.09% |
| 4 | polygalacturonase, pectin methyl esterase, pectin lyase, arabinofuranosidase, cellulase and xylanase + rhamnogalacturonase and mannanase | 125 ppm + 125 ppm | 5.70% |

## Example 4

*Yield increase of olive oil production by using enzymes*

Olives of maturity index (MI) 2.5 were crushed into olive paste in a crusher. The enzyme solution was added into the crusher during the entire crushing process.

One ton of crused olive paste was incubated with and without an enzyme composition under slow stirring at 25°C for 30 minutes in a malaxer. In experiment 1, no enzymes were used; in experiment 2, 250 ppm of an enzyme composition comprising a polygalacturonase, a pectin methyl esterase, a pectin lyase, an arabinofuranosidase, a cellulase and a xylanase was used; in experiment 3, 250 ppm of an enzyme composition comprising a beta-glucanase was used; in experiment 4, 200 ppm of an enzyme composition comprising a polygalacturonase, a pectin methyl esterase, a pectin lyase, an arabinofuranosidase, a cellulase and a xylanase and 50 ppm of an enzyme composition comprising a beta-glucanase was used.

After incubation, the olive paste went into a decanter to separate the crude oil from the pomance, and the obtained crude oil went through a disc centrifuge to remove the water in the crude oil to obtain final olive oil product. During the decantation process, pomace was collected to measure the oil content by Near Infrared Reflectance (NIR), which was used as indicator of oil yield. The lower the oil content in pomace, the higher the oil yield in the crude olive oil will be.

The results are shown in Table 4. Table 4 clearly demonstrates that there is a higher yield of olive oil when olive oil is produced by using a polygalacturonase, a pectin methyl esterase, a pectin lyase, an arabinofuranosidase, a cellulase, a xylanase and a beta-glucanase compared to when olive oil is produced by using a polygalacturonase, a pectin methyl esterase, a pectin lyase, an arabinofuranosidase, a cellulase and a xylanase or when olive oil is produced by using a cellulase and a beta-glucanase.

Table 4: Remaining olive oil in pomace after enzymatic treatment.

| Experiment number | Enzyme composition constituents | Dosage | Oil content in pomace |
|---|---|---|---|
| 1 | - | - | 13.12% |
| 2 | polygalacturonase, pectin methyl esterase, pectin lyase, arabinofuranosidase, cellulase and xylanase | 250 ppm | 6.06% |
| 3 | beta-glucanase | 250 ppm | 6.12% |

(continued)

| Experiment number | Enzyme composition constituents | Dosage | Oil content in pomace |
|---|---|---|---|
| 4 | polygalacturonase, pectin methyl esterase, pectin lyase, arabinofuranosidase, cellulase and xylanase + beta-glucanase | 200 ppm + 50 ppm | 6.00% |

**Claims**

1. A process for producing olive oil comprising the step of adding enzymes to olive paste and incubating the obtained mixture, wherein the enzymes comprise a polygalacturonase, a pectin methyl esterase, a pectin lyase, an arabinofuranosidase, a cellulase, a xylanase, a rhamnogalacturonase and a mannanase.

2. The process according to claim 1, wherein the enzymes further comprise a beta-glucanase.

3. The process according to claim 1 and 2, wherein the enzymes are added during and/or after crushing olives into olive paste.

4. The process according to any one of the claims 1 to 3, wherein the olive paste is made from olives having a maturity index of 0 or 1.

5. The process according to any one of the claims 1 to 3, wherein the olive paste is made from olives having a maturity index of 2 or 3.

6. The process according to any one of the claims 1 to 5, wherein the process further comprises subjecting the obtained mixture to malaxation.

7. The process according to any one of the claims 1 to 6, wherein the process further comprises a solid-liquid separation after malaxation.

8. The process according to claim 7, wherein the solid-liquid separation is performed by applying pressure, percolation and/or centrifugation techniques.

9. The process according to any one of the claims 1 to 8, wherein the enzymes are added in an amount ranging from 100 to 1000 ppm.

10. The process according to any one of the claims 1 to 9, wherein the obtained mixture is incubated for 10 to 200 minutes at a temperature ranging from 5°C to 70$^0$C.

11. The process according to any one of the claims 1 to 10, wherein the obtained mixture is incubated in a container having a volume ranging from 0.5 $m^3$ to 5000 $m^3$.

12. The process according to any one of the claims 1 to 11, wherein the enzymes are liquid.

13. An olive paste comprising a polygalacturonase, a pectin methyl esterase, a pectin lyase, an arabinofuranosidase, a cellulase, a xylanase, a rhamnogalacturonase, a mannanase and optionally a beta-glucanase.

**Patentansprüche**

1. Verfahren zur Herstellung von Olivenöl, umfassend den Schritt, bei dem Olivenpaste mit Enzymen versetzt und das erhaltene Gemisch inkubiert wird, wobei die Enzyme eine Polygalacturonase, eine Pektin-Methylesterase, eine Pektin-Lyase, eine Arabinofuranosidase, eine Cellulase, eine Xylanase, eine Rhamnogalacturonase und eine Mannanase umfassen.

2. Verfahren nach Anspruch 1, wobei die Enzyme ferner eine Beta-Glucanase umfassen.

3. Verfahren nach Anspruch 1 und 2, wobei Enzyme während und/oder nach dem Zerkleinern von Oliven zu Olivenpaste zugesetzt werden.

4. Verfahren nach einem der Ansprüche 1 bis 3, wobei die Olivenpaste aus Oliven mit einem Reifeindex von 0 oder 1 hergestellt wird.

5. Verfahren nach einem der Ansprüche 1 bis 3, wobei die Olivenpaste aus Oliven mit einem Reifeindex von 2 oder 3 hergestellt wird.

6. Verfahren nach einem der Ansprüche 1 bis 5, wobei das Verfahren ferner Malaxieren des erhaltenen Gemischs umfasst.

7. Verfahren nach einem der Ansprüche 1 bis 6, wobei das Verfahren ferner eine Trennung von Feststoff und Flüssigkeit nach dem Malaxieren umfasst.

8. Verfahren nach Anspruch 7, wobei die Trennung von Feststoff und Flüssigkeit durch Anlegen von Druck, Perkolations- und/oder Zentrifugationstechniken erfolgt.

9. Verfahren nach einem der Ansprüche 1 bis 8, wobei die Enzyme in einer Menge im Bereich von 100 bis 1000 ppm zugesetzt werden.

10. Verfahren nach einem der Ansprüche 1 bis 9, wobei das erhaltene Gemisch 10 bis 200 Minuten bei einer Temperatur im Bereich von 5°C bis 70°C inkubiert wird.

11. Verfahren nach einem der Ansprüche 1 bis 10, wobei das erhaltene Gemisch in einem Behälter mit einem Volumen im Bereich von 0,5 m$^3$ bis 5000 m$^3$ inkubiert wird.

12. Verfahren nach einem der Ansprüche 1 bis 11, wobei die Enzyme als Flüssigkeit vorliegen.

13. Olivenpaste, umfassend eine Polygalacturonase, eine Pektin-Methylesterase, eine Pektin-Lyase, eine Arabinofuranosidase, eine Cellulase, eine Xylanase, eine Rhamnogalacturonase, eine Mannanase und gegebenenfalls eine Beta-Glucanase.

## Revendications

1. Procédé pour la production d'huile d'olive comprenant l'étape d'ajout d'enzymes à une pâte d'olives et l'incubation du mélange obtenu, les enzymes comprenant une polygalacturonase, une pectine méthyle estérase, une pectine lyase, une arabinofuranosidase, une cellulase, une xylanase, une rhamnogalacturonase et une mannanase.

2. Procédé selon la revendication 1, les enzymes comprenant en outre une bêta-glucanase.

3. Procédé selon les revendications 1 et 2, les enzymes étant ajoutées pendant et/ou après le broyage d'olives en une pâte d'olives.

4. Procédé selon l'une quelconque des revendications 1 à 3, la pâte d'olives étant préparée à partir d'olives ayant un indice de maturité de 0 ou 1.

5. Procédé selon l'une quelconque des revendications 1 à 3, la pâte d'olives étant préparée à partir d'olives ayant un indice de maturité de 2 ou 3.

6. Procédé selon l'une quelconque des revendications 1 à 5, le procédé comprenant en outre la soumission du mélange obtenu à un malaxage.

7. Procédé selon l'une quelconque des revendications 1 à 6, le procédé comprenant en outre une séparation solide-liquide après malaxage.

8. Procédé selon la revendication 7, la séparation solide-liquide étant réalisée en appliquant des techniques de pression, de percolation et/ou de centrifugation.

9. Procédé selon l'une quelconque des revendications 1 à 8, les enzymes étant ajoutées en une quantité dans la plage de 100 à 1 000 ppm.

10. Procédé selon l'une quelconque des revendications 1 à 9, le mélange obtenu étant incubé pendant 10 à 200 minutes à une température dans la plage de 5 °C à 70 °C.

11. Procédé selon l'une quelconque des revendications 1 à 10, le mélange obtenu étant incubé dans un récipient ayant un volume dans la plage de 0,5 m$^3$ à 5 000 m$^3$.

12. Procédé selon l'une quelconque des revendications 1 à 11, les enzymes étant liquides.

13. Pâte d'olives comprenant une polygalacturonase, une pectine méthyle estérase, une pectine lyase, une arabinofuranosidase, une cellulase, une xylanase, une rhamnogalacturonase, une mannanase et éventuellement une bêta-glucanase.

**REFERENCES CITED IN THE DESCRIPTION**

**Patent documents cited in the description**

- DE 10339010 A1
- WO 2006114094 A
- WO 2009073383 A